# EUROPÄISCHE PATENTSCHRIFT

(11) **EP 1 920 726 B1**
(45) Veröffentlichungstag und Bekanntmachung des Hinweises auf die Patenterteilung: **09.04.2014**
(21) Anmeldenummer: 07021846.6
(22) Anmeldetag: 09.11.2007
(51) Int. Cl.: A61B 18/14

(54) **Aktivelektrode für die HF-Chirurgie**
Active electrode for HF surgery
Electrode active pour la chirurgie HF

(30) Priorität: 10.11.2006 DE 102006053338
(43) Veröffentlichungstag der Anmeldung: 14.05.2008
(73) Patentinhaber: Steidle, Eduard, 78532 Tuttlingen (DE)
(72) Erfinder: Steidle, Eduard, 78532 Tuttlingen (DE)
(74) Vertreter: Weiss, Peter

(56) Entgegenhaltungen:
- DE-U1- 29 502 764
- US-A- 4 492 231
- US-A1- 2002 072 746

## Beschreibung

Die Erfindung betrifft eine Aktivelektrode für die HF-Chirurgie, insbesondere zum Schneiden und Koagulieren von Gewebe in der Human- und Veterinärmedizin.

Die Hochfrequenzchirurgie wird bereits seit geraumer Zeit sowohl in der Human- als auch in der Veterinärmedizin mit Erfolg eingesetzt, um menschliches oder tierisches Gewebe zu schneiden und/oder zu koagulieren, Dabei wird mit geeigneten elektrochirurgischen Instrumenten gearbeitet, die sich durch Umsetzung der Energie eines hochfrequenten elektromagnetischen Wechselfeldes derart erwärmen, dass damit ein biologisches Gewebe z.B. getrennt werden kann, wobei die hierbei auftretende Koagulation gleichzeitig blutstillend wirkt. Bekanntlich entsteht bei der Elektrokoagulation ein Funke, der eine punkt- oder strichförmige, tief reichende Verbrennung des zu behandelnden Gewebes bewirkt.

Zur Durchführung eines derartigen Schneidvorgangs und/oder einer Koagulation werden HF-Chirurgiegeräte verwendet, die mindestens eine an einem Elektrodenträger angeordnete Aktivelektrode zur Einführung in das Gewebe aufweisen, die aus Metall besteht, da Metall ganz allgemein ein elektrisch gut leitender und gut wärmeübertragender Stoff ist. Letzteres ist auch für eine Hochfrequenzerwärmung eines chirurgischen Instruments wichtig, da diese letzten Endes an dem Erfolg eines richtigen Gewebeschnittes mit gleichzeitiger blutstillender Koagulation maßgeblich beteiligt ist.

Das Dokument US 4 492 231 A offenbart ein nicht anhaftendes Elektrokauterisationssystem mit Elektrokauterisationspinzette, welche an einen HF-Generator angeschlossen ist.

Dieses Dokument offenbart den nächstliegenden Stand der Technik.

Das Dokument DE 295 02 764 U1 offenbart ein Instrument für die Elektrochirurgie, mit einem Grundkörper aus Keramik, welcher teilweise metallbeschichtet ist. Die Metallbeschichtung kann elektrisch leitend mit einem Steckerteil verbunden werden.

Das Dokument US 2002/072746 A1 offenbart ein chirurgisches Instrument zur Koagulation mit Kontaktflächen aus dotiertem Diamant zur Herstellung der elektrischen Leitfähigkeit.

Die vorliegende Erfindung hat sich daher die Aufgabe gestellt, die Aktivelektrode eines HF-Chirurgieinstruments dahingehend zu verbessern, dass sich die Schneidleistung der Elektrode bei einem Operationsvorgang erhöht und gleichzeitig eine einwandfreie Koagulation gewährleistet ist.

Die Erfindung ist Anspruch 1 definiert.

In der Praxis bewährt hat sich vor allem eine Legierung für die Aktivelektrode mit einer wesentlichen Zusammensetzung aus 60 % Ag und 40 % Cu bzw. 55 % Ag und 45 % Cu, wobei die zuletzt angegebene Zusammensetzung auch innerhalb der Bereiche 40 % - 60 % Ag und 40 % - 60 % Cu schwanken kann,

Der mit der höheren elektrischen Leitfähigkeit bzw. Wärmeableitfähigkeit ausgestattete Teil des Elektrodenkörpers ist vorteilhaft als eine Art messerartige Flachklinge ausgebildet, die annähernd parallel verlaufende Seitenflächen aufweist, wobei die Höhe der Flachklinge ein Mehrfaches ihrer Dicke betragen soll, wodurch sich die Wärmeableitfähigkeit erhöht und somit das chirurgische Instrument effektiver zum Einsatz kommen kann.

Ein wesentlicher Vorteil bei der vorliegenden Erfindung ist, dass durch den hohen Silberanteil die Aktivelektrode, insbesondere deren Seiten- und Schneideflächen beim Operieren nicht am Gewebe anhaften bzw. keine Gewebereste an diesen Flächen haften bleiben.

Zur Verbesserung der Schneidfähigkeit der Aktivelektrode ist es ferner besonders zweckmäßig, dass das freie Ende der Flachklinge abgeschrägt ausgebildet ist. Hierbei hat sich gezeigt, dass die Abschrägung zwischen 30 ° und 60 ° liegen sollte, wobei in der Praxis mit einer Abschrägung von 45 ° bestens gearbeitet werden konnte,

Schließlich kann die Schneidfähigkeit der Aktivelektrode auch dadurch noch verbessert werden, indem die Stirnfläche der Abschrägung in ihrer Draufsicht keilförmig ausgebildet wird.

Ferner hat sich auch bei der vorliegenden Erfindung als vorteilhaft erwiesen, den Elektrodenkörper der Aktivelektrode bzw. Flachelektrode durch mehrfaches Umformen oder Pressen zu behandeln, um die Steifigkeit der Aktivelektrode deutlich zu erhöhen. Gerade bei sehr weichen Edelmetallen wie Silber oder Legierungen aus Silber und Kupfer hat es sich zu diesem Zweck als besonders vorteilhaft erwiesen, durch Umformen oder Pressen die Steifigkeit, nahe der Streckgrenze derart zu erhöhen, so dass Festigkeit der Aktivelektrode deutlich verbessert ist.

Zudem hat sich als besonders vorteilhaft bei der vorliegenden Erfindung erwiesen, dass durch den sehr hohen Anteil von Silber, die Wärmeleitfähigkeit und Wärmeableitfähigkeit deutlich gesteigert werden kann, die bei Silber etwa 420 W/mK und Kupfer von etwa 200 W/mK beträgt. Im Vergleich zu Edelstahl beträgt die Wärmeleitfähigkeit 15 W/mK.

Somit ist bei sehr hohem Silberanteil die Wärmeleitfähigkeit und damit auch die Wärmeableitfähigkeit deutlich verbessert, was das Schneiden und gleichzeitige Koagulieren erheblich verbessert.

Weitere Merkmale und Vorteile der vorliegenden Erfindung ergeben sich aus der nachfolgenden Beschreibung eines Ausführungsbeispiels in Verbindung mit der Zeichnung; es zeigen:
- Fig. 1: eine vereinfachte perspektivische Ansicht eines Elektrodenhalters mit eingesetzter Aktivelektrode,
- Fig. 2: eine vergrößerte Seitenansicht der Aktivelektrode gemäß Fig. 1 und
- Fig. 3: den vorderen Abschnitt der Aktivelektrode gemäß Fig. 2 von unten gesehen.

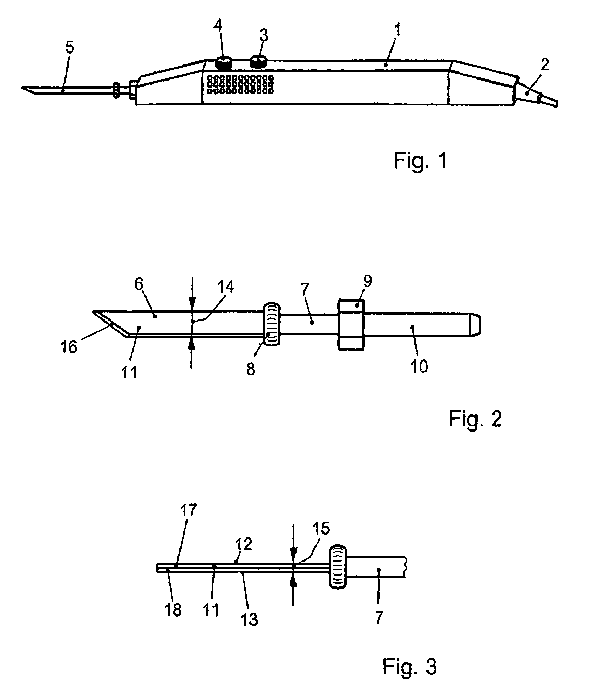

Der in Fig. 1 gezeigte Elektrodenhalter - bezeichnet mit 1 - besitzt einen Anschluss 2 für eine nicht dargestellte hochfrequente Wechselstromquelle, zwei Bedienungsknöpfe 3 und 4, sowie eine in den Elektrodenhalter eingesteckte Aktivelektrode 5, welche in Fig. 2 vergrößert im einzelnen dargestellt ist. Die Aktivelektrode 5 kann ein- oder mehrpolig betrieben werden.

Der Elektrodenhalter 1 selbst besteht aus Kunststoff, wogegen die Aktivelektrode 5 aus Metall gefertigt ist, und zwar das eigentliche als Flachelektrode 6 ausgebildete Schneidmesser aus einer Legierung aus 60 Gew. % Ag und 40 Gew, % Cu. Da Silber (Ag) gegenüber Kupfer (Cu) eine wesentlich höhere Wärmeleitfähigkeit bzw. Wärmeableitfähigkeit besitzt, ist es von Vorteil, den Anteil an Silber möglichst hoch zu wählen. Auf der anderen Seite leidet die Elektrode an Festigkeit, sodass je nach Anwendung ein geeignetes Mischungsverhältnis zwischen Silber und Kupfer gewählt werden muss. Die Flachelektrode 6 kann entweder vollkommen aus der oben angegebenen Legierung oder aus Ag/Cu hergestellt werden oder aber auch aus der besagten Legierung und aus einem weiteren Metall bestehen, wobei hier vor allem der Preis und die Festigkeit eine Rolle spielt. Die Aktivelektrode 5 kann zwar als Flachelektrode 6 ausgebildet sein, diese kann jedoch auch andere Formen aufweisen und ist im wesentlichen als grossflächig ausgebildeter Elektrodenkörper gebildet.

Im vorliegenden Ausführungsbeispiel ist die Flachelektrode 6 mit einem Halteteil 7 des übrigen Elektrodenkörpers verbunden, das hierzu einen Bund 8 aufweist, an den die Flachelektrode 6 durch einen Schmelzvorgang befestigt wurde. Ferner besitzt das Halteteil 7 noch einen Anschlagbund 9 an den sich ein Steckzapfen 10 anschließt mit dem die Aktivelektrode 5 in den Elektrodenhalter 1 gesteckt wird.

Die Flachelektrode 6 ist als eine Art Flachklinge 11 messerartig ausgebildet, und besitzt annähernd parallele Seitenflächen 12 und 13, wobei die mit 14 bezeichnet Höhe der Flachklinge 11 ein Mehrfaches ihrer mit 15 bezeichneten Dicke beträgt; im vorliegenden Ausführungsbeispiel Höhe/Dicke wie 4:1.

Am freien Ende ist die Flachklinge 11 mit einer Abschrägung 16 von 45 ° versehen, deren Stirnfläche in ihrer Draufsicht keilförmig ausgebildet ist, wobei die keilförmige Ausbildung zwei Schrägflächen 17 und 18 aufweist, welche insbesondere in Fig. 3 zu sehen sind.

Insgesamt ist mit der vorliegenden Erfindung eine hervorragende Hochfrequenzchirurgieelektrode für chirurgische Eingriffe geschaffen worden, die sich besonders durch ihren effektiven Einsatz bei hierfür geeigneten Operationen auszeichnet, und zwar aufgrund seiner material- und formbedingten Beschaffenheit, die einen relativ hohen Wärmeübergang mit sich bringt, der sowohl den Schneidvorgang selbst als auch eine ausreichende Koagulation begünstigt.

Ferner soll im Rahmen der vorliegenden Erfindung liegen, dass eine äussere Oberfläche zumindest teilweise, insbesondere vollständig mit einer Beschichtung, insbesondere Diamantbeschichtung versehen ist.

Dabei kann die Beschichtung bspw. als diamantartige Kohlenstoffbeschichtung auf die vollständige Oberfläche der Aktivelektrode 5 aufgetragen sein.

Damit noch eine Spannung auf die äussere Oberfläche angelegt werden kann, können der Beschichtung bspw. elektrisch leitende Partikel zugesetzt sein, wie bspw. aus Silber oder Kupfer,

Dabei kann auf die Aktivelektrode 5 die Beschichtung, insbesondere die elektrisch leitfähige Beschichtung in unterschiedlichen beliebigen Verfahren, ausgedampft, aufgetragen, mittels Hochfrequenzauftrag oder Polykristallwachstum etc, aufgetragen werden.

Dabei kann die Beschichtung in einer Dicke von etwa 0,1 bis 100 µm Dicke auf die Aktivelektrode 5 aufgetragen werden.

Von Vorteil ist, insbesondere bei diamantartigen Kohlenstoffbeschichtungen, dass eine Wärmeleitfähigkeit bzw. Wärmeableitfähigkeit noch deutlich gegenüber dem Silber erhöht ist, diese liegt in etwa bei 2000 W/mK.

Somit kann auch ein Koagulieren bei gleichzeitiger erhöhter Schnitt- und Kratzfestigkeit deutlich verbessert werden. Ein Ankleben an das Gewebe beim Koagulieren wird ebenfalls verhindert.

**Bezugszeichenliste**

| | | | | | |
|---|---|---|---|---|---|
| 1 | Elektrodenhalter | 34 | | 67 | |
| 2 | Anschluss | 35 | | 68 | |
| 3 | Bedienungsknopf | 36 | | 69 | |
| 4 | Bedienungsknopf | 37 | | 70 | |
| 5 | Aktivelektrode | 38 | | 71 | |
| 6 | Flachelektrode | 39 | | 72 | |
| 7 | Halteteil | 40 | | 73 | |
| 8 | Bund | 41 | | 74 | |
| 9 | Anschlagbund | 42 | | 75 | |
| 10 | Steckzapfen | 43 | | 76 | |
| 11 | Flachklinge | 44 | | 77 | |
| 12 | Seitenfläche | 45 | | 78 | |
| 13 | Seitenfläche | 46 | | 79 | |
| 14 | Höhe | 47 | | | |
| 15 | Breite | 48 | | | |
| 16 | Abschrägung | 49 | | | |
| 17 | Schrägfläche | 50 | | | |
| 18 | Schrägfläche | 51 | | | |
| 19 | | 52 | | | |
| 20 | | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Aktivelektrode für die HF-Chirurgie, insbesondere zum Schneiden und Koagulieren von Gewebe in der Human- und Veterinärmedizin,
wobei zumindest der mit der höheren elektrischen Leitfähigkeit und Wärmeableitfähigkeit ausgestattete Teil des Elektrodenkörpers (6), im wesentlichen aus einer Legierung mit 40 % - 98 % Silber und 2 % - 60 % Kupfer besteht, und zwar jeweils auf das Gewicht bezogen, **dadurch gekennzeichnet, dass** die Aktivelektrode (5), insbesondere dessen Elektrodenkörper (6) an ihrer äusseren Oberfläche eine diamantartige Kohlenstoffbeschichtung aufweist, wobei die diamantartige Kohlenstoffbeschichtung eine Dicke von 0,1 bis 100 µm aufweist.

2. Aktivelektrode nach Anspruch 1, **dadurch gekennzeichnet, dass** die diamantartige Kohlenstoffbeschichtung elektrisch leitende Partikel enthält und elektrisch leitend ausgebildet ist.

3. Aktivelektrode nach wenigstens einem der vorangehenden Ansprüche, **dadurch gekennzeichnet, dass** durch die diamantartige Kohlenstoffbeschichtung eine Wärmeleitfähigkeit und Wärmeableitfähigkeit im Oberflächenbereich deutlich erhöht ist.

4. Aktivelektrode nach Anspruch 1, **dadurch gekennzeichnet, dass** der wesentliche Teil des Elektrodenkörpers einer Aktivelektrode (5) als Flachelektrode (6) oder als grossflächig ausgebildeter Elektrodenkörper ausgebildet ist, deren zumindest mit dem zu behandelnden Gewebe in Kontakt kommenden Flächen(12, 13, 17, 18) eine höhere elektrische Leitfähigkeit bzw. Wärmeableitfähigkeit aufweisen.

5. Aktivelektrode nach Anspruch 1 oder 4, **gekennzeichnet durch** eine das Zusammensetzung der Legierung aus im wesentlichen 40 % - 80 %, insbesondere 60 % Silber und 20 % - 60 %, insbesondere 40 % Kupfer.

6. Aktivelektrode nach wenigstens einem der Ansprüche 1 oder 5, **gekennzeichnet durch** eine Zusammensetzung der Legierung aus im wesentlichen 40 % - 60 %, insbesondere 55 % Silber und 40 % - 60 %, insbesondere 45 % Kupfer.

7. Aktivelektrode nach wenigstens einem der vorangehenden Ansprüche, **dadurch gekennzeichnet, dass** der mit der höheren elektrischen Leitfähigkeit bzw. Wärmeableitfähigkeit ausgestattete Teil des Elektrodenkörpers (6) als eine Art Flachklinge (11) oder nadelartig oder kugelartig ausgebildet ist.

8. Aktivelektrode nach Anspruch 7, **dadurch gekennzeichnet, dass** die Flachklinge (11) messerartig mit annähernd parallelen Seitenflächen (12, 13) ausgebildet ist.

9. Aktivelektrode nach Anspruch 7 oder 8, **dadurch gekennzeichnet, dass** die Höhe (14) der Flachklinge (11) ein Mehrfaches ihrer Dicke (15) beträgt.

10. Aktivelektrode nach einem der Ansprüche 7 bis 9, **dadurch gekennzeichnet, dass** das freie Ende der Flachklinge (11) abgeschrägt ausgebildet ist.

11. Aktivelektrode nach Anspruch 10, **dadurch gekennzeichnet, dass** die Abschrägung (16) 80 - 60 °, insbesondere 45 ° beträgt.

12. Aktivelektrode nach Anspruch 10, **dadurch gekennzeichnet, dass** die Stirnfläche der Abschrägung (16) in ihrer Draufsicht keilförmig (17, 18) ausgebildet ist.

13. Aktivelektrode nach wenigstens einem der vorangehenden Ansprüche, **dadurch gekennzeichnet, dass** eine Festigkeit der Aktivelektrode (5), insbesondere des Elektrodenkörpers (6) durch Umformen oder Pressen, insbesondere Kaltumformen steigerbar ist.

## Claims

1. An active electrode for HF surgery, in particular for cutting and coagulating tissue in human and veterinary medicine,
wherein at least that part of the electrode body (6) which is equipped with the greater electrical conductivity and heat dissipation ability consists substantially of an alloy with 40% - 98% silver and 2% - 60% copper, namely relative to weight in each case, **characterised in that** the active electrode (5), in particular the electrode body (6) thereof, has on its outer surface a diamond-like carbon coating, the diamond-like carbon coating having a thickness of 0.1 to 100 µm.

2. An active electrode according to Claim 1, **characterised in that** the diamond-like carbon coating contains electrically conductive particles and is designed to be electrically conductive.

3. An active electrode according to at least one of the preceding claims, **characterised in that** owing to the diamond-like carbon coating a thermal conductivity and heat dissipation ability in the surface region is considerably increased.

4. An active electrode according to Claim 1, **characterised in that** the fundamental part of the electrode body of an active electrode (5) is designed as a flat electrode (6) or as an electrode body formed with a large surface area, the surfaces (12, 13, 17, 18) of which which come into contact at least with the tissue to be treated have a greater electrical conductivity or heat dissipation ability.

5. An active electrode according to Claim 1 or 4, **characterised by** a composition of the alloy of substantially 40% - 80%, in particular 60%, silver and 20% - 60%, in particular 40%, copper.

6. An active electrode according to at least one of Claims 1 or 5,
**characterised by** a composition of the alloy consisting of substantially 40% - 60%, in particular 55%, silver and 40% - 60%, in particular 45%, copper.

7. An active electrode according to at least one of the preceding claims, **characterised in that** that part of the electrode body (6) which is equipped with the greater electrical conductivity or heat dissipation ability is designed as a type of flat blade (11) or in needle-like or sphere-like manner.

8. An active electrode according to Claim 7, **characterised in that** the flat blade (11) is designed in a knife-like manner with approximately parallel lateral faces (12, 13).

9. An active electrode according to Claim 7 or 8, **characterised in that** the height (14) of the flat blade (11) is a multiple of its thickness (15).

10. An active electrode according to one of Claims 7 to 9, **characterised in that** the free end of the flat blade (11) is formed bevelled.

11. An active electrode according to Claim 10, **characterised in that** the bevelling (16) is 30 - 60°, in particular 45°.

12. An active electrode according to Claim 10, **characterised in that** the end face of the bevelling (16) is designed to be wedge-shaped (17, 18) in its top view.

13. An active electrode according to at least one of the preceding claims, **characterised in that** a strength of the active electrode (5), in particular of the electrode body (6), can be increased by shaping or pressing, in particular cold-shaping.

## Revendications

1. Electrode active pour la chirurgie HF, en particulier pour la découpe et la coagulation de tissus dans la médecine humaine et vétérinaire,
dans laquelle au moins la partie du corps d'électrode (6) équipée de la conductivité électrique et de l'aptitude à la dissipation de chaleur supérieures, est réalisée substantiellement en un alliage avec de 40 % à 98 % d'argent et de 2% à 60 % de cuivre, et ce chaque fois par rapport au poids,
**caractérisé en ce**
**que** l'électrode active (5), en particulier le corps d'électrode (6) présente sur sa surface extérieure un revêtement de carbone semblable au diamant, le revêtement de carbone semblable au diamant présentant une épaisseur de 0,1 à 100 µm.

2. Electrode active selon la revendication 1, **caractérisée en ce que** le revêtement de carbone semblable au diamant contient des particules électro-conductrices, et est réalisé électro-conducteur.

3. Electrode active selon au moins l'une des revendications précédentes, **caractérisée en ce que** par le revêtement de carbone semblable au diamant la conductivité thermique et l'aptitude à la dissipation de la chaleur dans la zone de surface sont nettement augmentées.

4. Electrode active selon la revendication 1, **caractérisée en ce que** la partie essentielle du corps d'électrode d'une électrode active (5) est réalisée sous forme d'électrode plate (6) ou de corps d'électrode à grande surface dont au moins les surfaces (12, 13, 17 18) entrant en contact avec le tissu à traiter présentent une conductivité électrique ou aptitude à la dissipation de chaleur plus élevée.

5. Electrode active selon la revendication 1 ou 4, **caractérisée par** une composition de l'alliage de substantiellement 40 % à 80 %, en particulier 60 % d'argent et de 20 % à 60 %, en particulier de 40% de cuivre.

6. Electrode active selon au moins l'une des revendications 1 ou 5, **caractérisée par** une composition de l'alliage de substantiellement 40 % à 60 %, en particulier 55 % d'argent et de 40 % à 60 %, en particulier de 45% de cuivre.

7. Electrode active selon au moins l'une des revendications précédentes, **caractérisée en ce que** la partie du corps d'électrode (6) équipée de la conductivité électrique ou de l'aptitude à la dissipation de chaleur plus élevée est réalisée sous forme d'une sorte de lame plate (11) ou en forme d'aiguille ou en forme de bille.

8. Electrode active selon la revendication 7, **caractérisée en ce que** la lame plate (11) est réalisée en forme de couteau à faces latérales (12, 13) environ parallèles.

9. Electrode active selon la revendication 7 ou 8, **caractérisée en ce que** la hauteur (14) de la lame plate (11) est un multiple de son épaisseur (15).

10. Electrode active selon l'une des revendications 7 à 9, **caractérisée en ce que** l'extrémité libre de la lame plate (11) est réalisée à chanfrein.

11. Electrode active selon la revendication 10, **caractérisée en ce que** le chanfrein (16) est de 30 à 60°, en particulier de 45°.

12. Electrode active selon la revendication 10, **caractérisée en ce que** la surface frontale du chanfrein (16) est réalisée, en vue en plan, en forme de coin (17, 18).

13. Electrode active selon au moins l'une des revendications précédentes, **caractérisée en ce qu'**une solidité de l'électrode active (5), en particulier du corps d'électrode (6), est être augmentée par mise en forme ou pressage, en particulier par mise en forme à froid.
